(19) Europäisches Patentamt
European Patent Office
Office européen des brevets

(11) **EP 4 222 421 B1**

(12) **EUROPEAN PATENT SPECIFICATION**

(45) Date of publication and mention
of the grant of the patent:
**21.08.2024 Bulletin 2024/34**

(21) Application number: **21782720.3**

(22) Date of filing: **22.09.2021**

(51) International Patent Classification (IPC):
**F24F 8/20** *(2021.01)*

(52) Cooperative Patent Classification (CPC):
**F24F 8/20**

(86) International application number:
**PCT/EP2021/076096**

(87) International publication number:
**WO 2022/069320 (07.04.2022 Gazette 2022/14)**

(54) **ROTATIONAL DISINFECTANT DEVICE AND METHOD FOR CAPTURING AND DISINFECTING INFECTANTS FROM A GAS**

ROTIERENDE DESINFEKTIONSVORRICHTUNG UND VERFAHREN ZUR ERFASSUNG UND DESINFEKTION VON INFIZIERENDEN STOFFEN AUS EINEM GAS

DISPOSITIF DE DÉSINFECTION ROTATIF ET PROCÉDÉ POUR CAPTURER ET DÉSINFECTER DES AGENTS INFECTIEUX À PARTIR D'UN GAZ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2020 NL 2026584**

(43) Date of publication of application:
**09.08.2023 Bulletin 2023/32**

(73) Proprietor: **Romico Hold. VBA**
**6226 GV Maastricht (NL)**

(72) Inventor: **BROUWERS, Jozef Johannes Hubertus**
**3620 Lanaken (BE)**

(74) Representative: **Arnold & Siedsma**
**Bezuidenhoutseweg 57**
**2594 AC The Hague (NL)**

(56) References cited:
**EP-A1- 3 624 923      EP-B1- 3 624 923**
**KR-B1- 102 144 259    US-A- 2 662 759**
**US-A- 3 486 743**

**Description**

TECHNICAL FIELD

The present invention concerns a rotational disinfectant device for capturing and disinfecting infectants, comprising viruses and bacteria, from a gas, including air and gases produced in laboratories, factories and living organisms. The invention further relates to a method for capturing and disinfecting said infectant from a gas. The device and method are in particular suitable for capturing and disinfecting SARS-CoV-2 viruses from a gas, in particular from air.

BACKGROUND ART

Several epidemic illnesses are caused by viruses. COVID-19 for instance is caused by infection with the severe acute respiratory syndrome coronavirus 2 (SARS-CoV-2) virus strain. Transmission of viral diseases such as COVID-19 may be caused by cough- and/or sneeze respiratory droplets in a gas.

COVID-19 is a new disease, and many of the details of its spread are under investigation at present. This is one of the reasons that prior art relating to methods and devices configured to decrease spread of the SARS-CoV-2 virus is sparse. COVID-19 may spread from person to person, via several different modes, in particular when people are in close proximity to one another. Spread may occur very easily and sustainably, with one infected person possibly infecting more than one other person. It may transmit when people are symptomatic, but also when people do not show any or only minor symptoms.

The World Health Organisation (WHO) recommends at least 1 metre of social distance to hinder transmission, in combination with wearing face masks and washing hands, but people increasingly have problems with this behavior.

Viral diseases in particular spread by means of small droplets in the breath, usually referred to as aerosols or bio-aerosols. An aerosol is a suspension of fine solid particles or liquid droplets in a gas or in air. The liquid or solid particles have diameters that are typically around or less than 1 $\mu$m. As an example, SARS-CoV-2 virus particles are generally about 0.05 $\mu$m in size, but they are contained in larger saliva particles. Bacteria are typically 1 $\mu$m in size, or somewhat larger. The saliva particles and bacteria form an aerosol in air. When a person emits breath by coughing, sneezing and the like, the virus-containing saliva particles are emitted in the air where they evaporate rather easily, such as in about 1 sec, to yield smaller salt particles of about 1 $\mu$m that carry the virus particles. Also animals may produce such virus-containing particles as well as bacteria.

Problematic with aerosols is that the infectant particles contained in them may float for quite a long time over quite some distance, thereby increasing the risk for transmission of the disease associated with the infectant. For instance, it has been shown that SARS-CoV-2 particles of about 1 $\mu$m will fall in air under the influence of gravity forces for about 10 cm only after 1 hour of floating. Larger particles do not have this problem and particles of say 10 $\mu$m in size will fall in air at least a hundred times faster. When the virus-containing droplets eventually fall to floors or surfaces, they may remain infectious if people touch contaminated surfaces and then their eyes, nose or mouth with unwashed hands.

Airborne transmission of lower size aerosols occurs particularly in crowded and less ventilated indoor spaces, which are particularly effective for transmitting the virus, such as in restaurants, nightclubs, public transport and gatherings such as funerals. It also can occur in a healthcare setting, where certain medical procedures performed on COVID-19 patients generate aerosols.

It would be highly desirable to be able to prevent viruses from spreading, in particular in such potentially crowded and less ventilated indoor spaces. It is an aim of the present invention therefore to provide a disinfectant device for capturing and disinfecting infectants, comprising viruses, such as SARS-CoV-2, and bacteria, from a gas in order to prevent their spread. It is a further aim to provide a method for capturing and disinfecting such infectants from a gas. Yet a further aim is to disinfect infectants, such as bacteria, viruses, and particles containing bacteria and viruses, occurring in gases produced in laboratories, living organisms and industrial processes. EP3624923A1 discloses a rotational absorber device for scrubbing an absorbate from a gas and a method for scrubbing an absorbate from a gas.

DISCLOSURE OF THE INVENTION

These and other aims are provided by a device in accordance with claim 1. The invention provides a rotational disinfectant device for capturing and disinfecting infectants comprising viruses and bacteria from a gas, the device comprising a means for transmitting the infectant-containing gas to a gas inlet of a housing, which housing is further provided with a gas outlet, a disinfectant liquid inlet and a disinfectant liquid outlet; a rotor mounted for rotation in said housing and connecting to the inlets and outlets, the rotor comprising a plurality of disinfecting channels extending axially and parallel to a common rotation axis; and means for rotating the rotor. The channels are circumferentially enclosed by walls over an, optionally entire, axial length of the rotor between the disinfectant liquid inlet and the disinfectant liquid outlet; and the device further comprises means for providing a sustained flow of disinfectant liquid to the disinfectant liquid inlet.

The invented device allows to efficiently capturing and disinfecting infectants such as SARS-CoV-2 from a gas. Also bacteria present in the gas may be effectively removed by the device. It is to be understood that when infectants are mentioned in the present application, viruses and bacteria are included. Thereto, a method in accordance with the invention aimed at capturing and disinfecting infectants, such as SARS-CoV-2, from a gas comprises providing a rotational disinfectant device in accordance with the invention, transmitting and feeding infectant-containing gas to the gas inlet of the housing, feeding a disinfectant liquid to the disinfectant liquid inlet, rotating the rotor in said housing which causes the disinfectant liquid to confine to an inward facing wall of the disinfecting channels and form a film thereto, allowing transport of infectant particles from the gas outward to the disinfectant liquid in each disinfectant channel, and exiting the gas through the gas outlet and the disinfectant liquid that contains disinfected infectant particles through the disinfectant liquid outlet.

The rotational disinfectant device is able to capture and disinfect the infectant for at least 95% to about 99% of the infectant particles. In an improved embodiment of the invention, a high-efficiency particulate air (HEPA) filter may be positioned downstream of the rotational disinfecting device. Such a HEPA filter is able to capture infectant particles of typically lower than 0.1 $\mu$m from the gas, however is not able to disinfect such particles. The combination of the rotational disinfecting device and at least one HEPA filter positioned behind it significantly increases the useful life or standing time of the HEPA filter, the majority of the infectant particles being already captured and disinfected by the rotational disinfectant device. The risk for infection is also reduced considerably.

In useful embodiments, the high-efficiency particulate air (HEPA) filter is positioned downstream of the rotor of the rotational disinfectant device; or .the high-efficiency particulate air (HEPA) filter is positioned downstream of the disinfectant liquid collector of the rotational disinfectant device. Combinations may also be used.

The gas that enters the housing and the disinfecting channels of the rotating rotor contains a plurality of infectant particles, such as SARS-CoV-2 virus particles, and/or bacteria. Due to the centrifugal forces in the disinfecting channels, the infectant particles are transported to the disinfectant liquid that is confined against the radially inward facing walls of the channels. In a typical operation, the disinfectant liquid film that is formed on the channel walls absorbs a substantial part of the infectant particles, and this infectant particles-containing film in an embodiment flows parallel to the gas in the disinfecting channels and eventually breaks up in droplets when leaving the channels. Due to the rotation of the rotor, the disinfectant liquid droplets that contain (by now) disinfected (or killed) infectant particles, are propelled radially outwards with respect to a central axis of rotation of the rotor towards the disinfectant liquid outlet of the housing where the liquid is discarded and/or collected.

The invented rotational disinfectant device is not only able to capture infectant particles from the gas but at the same time kills the infectant, before the infectant even exits the device in the disinfectant liquid stream. A further advantage of the invented device is that it may be designed relatively small in size. This makes it possible to provide a plurality of invented devices relatively close to a source of infectant particles, in particular a human being. It has turned out that capturing infectant particles as soon as possible from the gas substantially lowers the transmission rate of a disease based on the infectant.

The present invention has a major advantage over the filter media commonly used for separating particles from gases such as air. Particles are entrapped in the pores of or on a surface of such filter media and accumulate there to a relatively high concentration on a relatively small area. Viruses and bacteria then remain active as a source of infection, and act as a kind of 'Corona bomb'. The present invention solves this problem fundamentally. Indeed, infectious particles, such as virus particles and/or bacteria, are centrifuged inside the rotating channels of the device towards and into a disinfecting film of disinfectant liquid. As a consequence, the infectious particles are disinfected and the hazard of infection by the collected disinfected particles is eliminated.

The small size of the invented device is achieved by providing the plurality of preferably parallel channels of small size in the rotor. The cross-sectional size of the disinfecting channels may be selected within a large range, depending on the specific application. Suitable cross-sectional widths of the channels may be as small as 1 mm, or even 0.1 mm, and preferably are limited to 10 mm, more preferably to 5 mm. Because of rotation of the rotor, the disinfectant liquid will migrate towards and eventually be confined to an inward facing wall of each channel, where it forms a film with a thickness that may be as small as 0.001 to 1 mm, more preferably between 0.010 and 0.1 mm. The small cross-sectional width of the channels and the small thickness of the film promote a low resistance to transport of the infectant particles from a gas to disinfectant liquid.

A further advantageous effect of the rotating rotor is that the disinfectant liquid is at least partly separated in the form of droplets that are propelled towards the liquid outlet. This rotation caused separation avoids having to install a separate liquid separator downstream of the actual disinfectant device.

Due to the action of the centrifugal force, liquid and gas that are brought into rotation when passed through a disinfecting channel of the rotor move radially away from the axis of rotation towards a radial boundary which extends parallel to the axis of rotation and forms a boundary of the channels in the rotor. The outer boundary of the disinfecting channels serves as a means for collecting those liquid parts that were able to reach and settle at this boundary and which can subsequently be removed from the gas flowing parallel along the collecting boundary. The extent to which liquid can reach the collecting boundary and act to absorb the infectant particles in the gas can be assessed from the time for a infectant particle to reach the collecting boundary in combination with the residence time, the time for the gas to pass through the channel. One skilled in the art will be able to adopt a suitable axial velocity of the gas in order to achieve the desired effect of liquid film forming and absorption of gas constituents. A suitable axial extent of the rotor and the disinfecting channels may be as large as 100-5000 times the cross-sectional width of the channels. For instance, a rotor of 20 cm long and 15 cm in diameter may be able to take in about 150 $m^3$/hr of infectant-containing gas. In a confined space of say 7-8 $m^3$ (which is typical for train compartments or busses for instance), an invented device would be able to refresh and disinfect the gas about each 3 min. Breathing air from humans amounts to about 0,5 $m^3$/hr. A rotor as small as 10 cm in length and 1.5 cm in diameter would be able to free this amount of air from virus-particles and/or bacteria.

The gas and the liquid in an embodiment of the rotational disinfectant device are inserted upstream of the rotor, whereto the device is provided with a gas inlet and an disinfectant inlet both located upstream of the rotor. The gas and the disinfectant liquid in this embodiment flow in the same direction, i.e. exhibit co-current flow. It may also be possible to define the the flow direction of the gas in relation to the liquid differently. In an embodiment of counter-current flow, the disinfectant liquid and the to-be-cleaned gas flow in opposite directions. To this end, the gas inlet and the disinfectant outlet of the rotational disinfectant device are located downstream of the rotor, while the disinfectant inlet is located upstream of the rotor. The main advantage of counter-current flow disinfection is that the cleaner the gas becomes - less infectant particles -, the lower the concentration of infectant particles in the disinfectant liquid will be. This provides an improved efficiency of disinfecting.

The amount of disinfecting channels may be chosen within a wide range, depending on the application. A suitable embodiment of the rotational disinfectant device comprises an amount of disinfecting channels of at least 10, more preferably at least 100, and most preferably at least 1000. Increasing the amount may increase efficiency at the expense of pressure drop across the rotor. Combining a large number of channels in one rotating rotor provides a means for handling large amounts of gas to be disinfected.

The channels may be provided in the rotor in any possible way, for instance by perforating a massive cylinder with the desired amount of channels, arranged parallel to the rotation axis of the cylinder. Another possibility is to provide a plurality of narrowly spaced concentric cylinders and each annulus between two adjacent cylinders is divided by at least one azimuthally placed axially extending partition. Other means of making the rotor may also be envisaged. For instance, a particular advantageous embodiment provides a rotational disinfectant device, wherein the rotor and/or the housing is made from a polymer, such as a polyolefin polymer, preferably a flame retardant polymer. Such a rotor and/or housing may conveniently be made by extrusion or pultrusion for instance. Suitable flame retardants may be chosen by one skilled in the art, depending on the specific requirements.

The channels are entirely circumferentially enclosed by walls over the entire axial length of the rotor between the disinfectant liquid inlet and outlet. Absence of holes or openings in the channel walls effectively prevents disinfectant liquid of leaking in a radial direction due to the action of the centrifugal force. It supports maintaining a homogeneous distribution of disinfectant liquid over the whole length of the rotating element. At the bottom (downstream) end, the centrifugal force acts as a means to propel disinfectant liquid radially upon leaving the rotating element or rotor. Liquid loaded by disinfected infectant particles is thus effectively separated from the gas which, depending on the way of operation, enters or leaves the rotating element or rotor at the bottom (downstream) end.

The means for providing a sustained flow of disinfectant liquid to the disinfectant liquid inlet may comprise a pump, or may be provided by the water supply system for instance, in case the disinfectant liquid comprises water.

The introduced disinfectant fluid develops a fluid film at the collecting boundary of each disinfecting channel. If the rotating rotor is installed with its axis of rotation vertically, collected fluid will preferably not flow downwards as a result of gravity but will preferably be entrained by the gas flow in the disinfecting channels due to shear forces. The level of shear force in the channels can be influenced by the pressure gradient in the disinfecting channels. This pressure gradient may be determined by the pressure of the upstream gas, i.e. the pressure of the gas at the gas inlet.

According to an embodiment of the invention, a rotational disinfectant device is provided wherein the gas transmitting means are located opposite to the gas inlet relative to the rotor. The gas transmitting means may conveniently comprise a propeller, or other device that is able to suction in environmental or gas in an efficient way. Another embodiment of the invention may relate to a device which further comprises pressurizing means for the inlet (fed) gas. In a corresponding embodiment of the method, the inlet (fed) gas is pressurized to a pressure of between 1 and 20 bar, more preferably of between 1 and 10 bar for best results.

Due to the centrifugal force, the liquid when leaving the rotor and the disinfecting channels thereof is propelled outwards. The rotation as such may not have a large effect on molecular transport and absorption. The rotation of the rotor however tends to hold the formed liquid film against channel walls and hinders or prevents this film from leaving said walls. The result is that apart from the absorption achieved, a good separation of gas and liquid is inherent to the absorption device. The liquid when exiting the channels therefore is well separated from the gas stream and can be collected. In the housing, means can be provided for continuous transportation of the disinfectant fluid.

The channels are preferably provided substantially parallel to the axis of rotation. Application of collecting walls positioned non-parallel to the axis of rotation results in a component of the centrifugal force acting parallel to the collecting boundary and can serve as a means for or to enhance the continuous transportation of the collected fluid film along the channel walls. However, such inclined walls may cause secondary flows, in particular under laminar flow conditions, such as secondary flows due to Coriolis forces. These secondary forces may disturb the process of radial migration and settling of liquid particles. A small inclination in the order of 0.1-1 radians may be beneficial.

An embodiment of the rotational disinfectant device further comprises flow direction means provided upstream and/or downstream of the rotor, whereby the optionally upstream flow direction means act as means for rotating the rotor.

Suitable embodiments of the rotational disinfectant device have flow direction means comprising a volute, a stator blade or an impeller, or a combination of these. These may bring the inlet gas in rotation and minimize pressure losses over the device, in particular when providing driven flow direction means installed upstream and/or downstream of the disinfecting channels of the rotor. The flow direction means may in some embodiments act as means for rotating the rotor.

In order to effectively collect the disinfectant liquid containing (part of) the disinfected infectants, the rotational disinfectant device further comprises a disinfectant liquid collector that connects to the disinfectant liquid outlet. This collector is preferably provided within the housing of the device and may for instance be embodied as circumferential ridge or tray, provided against a side wall of the housing. In said disinfectant liquid collector the collected particles settle to form a disinfected particle-rich bottom layer and a disinfected particle-poor top layer.

The rotational disinfectant device further comprises a return conduit that connects the disinfectant liquid collector outlet with the disinfectant liquid inlet, and a pump to transfer disinfectant liquid from the disinfected particle-poor top layer to the disinfectant liquid inlet for re-use. The return conduit, the liquid collector outlet and the disinfectant liquid inlet form a closed fluid circuit.

Yet another embodiment relates to a rotational disinfectant device, further comprising an outlet for the disinfected particle-rich bottom layer, optionally provided with a shut-off valve, which outlet is provided for draining the disinfected particle-rich bottom layer from the disinfectant liquid collector. In this way, the killed infectant particles may be discarded with.

Because of the relatively small cross-sectional width of the channels in the rotor, the gas within the channels may exert a rather strong shear force on the liquid film deposited on the boundary walls of the channels. This may cause the liquid to flow in the same direction as the gas stream. Gas and liquid then flow in the same direction and a further improvement of transfer of the infectant particles from the gas to the disinfectant liquid in the channels by such co-current flows may be achieved by preferred embodiments of the device in which rotors are provided in series. A suitable embodiment in this respect provides a rotational disinfectant device wherein the housing comprises a first and a second rotor both mounted for rotation in said housing whereby the second rotor is provided axially downstream from the first rotor.

The rotational disinfectant device may comprise a first and a second disinfectant liquid inlet, located upstream of the first and second rotor respectively, whereas another embodiment that may be combined with the first embodiment comprises a first and a second disinfectant liquid outlet, located downstream of the first and second rotor respectively.

At least one of the first and second disinfectant liquid outlets may also reconnect to at least one of the first and second disinfectant liquid inlets, and a device wherein the second disinfectant liquid outlet reconnects to the first disinfectant liquid inlet may also be used. In case of two rotors positioned in series with respect to each other, the gas exiting the first rotor enters the second rotor, while fresh disinfectant liquid is fed to the second rotor. When leaving the second rotor, the partly used disinfectant liquid is fed to the first rotor and leaves this rotor as more completely of even fully utilized disinfectant liquid.

In operation, the concentration of the infectant particles in the gas will reduce while the concentration of the disinfected particles in the liquid will increase. The equilibrium concentration in the gas, which corresponds to a certain concentration in the liquid, is determined by a number of factors comprising the temperature. The higher the temperature in the liquid, the higher the equilibrium concentration in the gas will be. Thus a reduction in temperature has a favourable effect on the yield of the rotational disinfectant device, which is preferably operated at temperatures below 50°C, more preferably below 40°C, and most preferably at room temperature.

Besides water, provided with a disinfectant, organic liquids may be used as disinfectant liquids. In appropriate cases chemicals or micro-organisms may be added to the disinfectant liquid for other purposes than to disinfect. Suitable disinfectants are known to one skilled in the art, such as chlorine and the like.

Although the centrifugally induced radial velocities of the fluid particles in the channels may be very small, small perturbations in the flow of the infectant-containing gas and liquid may be beneficial to the process of transporting the gaseous constituents as well as the liquid film formation at the outer boundary walls. An embodiment of the method wherein the hydraulic diameter of the disinfecting channels and the average axial gas velocity are selected such that the Reynolds number in the channels does not exceed 2000, more preferably 1800, and the gas passes through the disinfecting channels in a (potentially) laminar flow is preferred.. The Reynolds number is well known and depends on the average axial velocity of the gas flow through a channel, the kinematic viscosity of the carrier gas and the hydraulic diameter of the channel. The hydraulic diameter may be determined according to well known principles and, for a circular channel cross-section is equal to its diameter. The average axial velocity of the gas can be influenced by the pressure gradient over the disinfecting channels. Prevailing laminar flow in the disinfecting channels has been shown to improve the disinfectant efficiency.

Laminar flow in the channels may be achieved at a relatively moderate pressures difference across the rotor of below 5 bar and more preferably below 2 bar. Counter-current operation may then be possible by injecting liquid at the top (upstream end of the device) which flows downward while gas which is injected at the bottom (downstream end of the device) flows upward. A fan may be provided in order to impose the required pressure difference necessary to induce the upward gas flow. In this embodiment, clean gas leaves the rotor at the top and liquid loaded with killed infectant particles leaves at the bottom.

The rotational disinfectant device according to an embodiment of the invention is particularly useful when used as an assembly of a plurality of such rotational disinfectant devices. The assembly then preferably comprises a common vessel for disinfectant liquid, and the devices each connect to the common vessel for disinfectant liquid via a liquid conduct.

In another useful embodiment, the assembly comprises a pump means for pumping the disinfectant liquid to each rotational disinfectant device.

It has turned out that transmitting infectant-containing gas from a source such as a breathing human being as soon as possible to a rotational disinfectant device helps in reducing spread of the viral disease. An assembly according to an embodiment therefore is provided wherein each rotational disinfectant device is positioned in proximity of a source of infectant particles.

The assembly and device are particularly useful in disinfecting gas in a confined space that is frequented by a relatively large number of sources, i.e. breathing human beings. The invention therefore also relates to an indoor space, such as a restaurant or a public transport carriage, provided with a rotational disinfectant device or an assembly of such devices in accordance with embodiments of the invention.

It would be highly desirable to be able to provide a infectant-proof restaurant, bar or public transport carriage, such as a bus, a tram, or a train compartment. Such an aim is achieved by an embodiment of the invention that provides an indoor space provided with a plurality of seats, wherein each seat is associated with a rotational disinfectant device, optionally by providing a rotational disinfectant device under each such seat or in the back of the seat provided in front of the passenger. Emitted infectant particles are in such an embodiment effectively collected from the gas and transmitted and fed to the channels of each rotational disinfectant device, in which channels the infectant is killed by contacting it with the disinfectant liquid, in a relatively short time of a few seconds.

A useful method for capturing and disinfecting infectants comprising viruses, such as SARS-CoV-2, and bacteria, from a gas makes use of one or more of the invented rotational disinfectant devices. A method comprises providing one or more of such rotational disinfectant devices, transmitting and feeding the infectant-containing gas to the gas inlet of the housing of such device, feeding a disinfectant liquid to the disinfectant liquid inlet, rotating the rotor in said housing which causes the disinfectant liquid to confine to an inward facing wall of the disinfecting channels and form a film thereto, allowing transport of infectant particles from the gas to the disinfectant liquid, and exiting the gas through the gas outlet and the infectant particle-containing disinfectant liquid through the disinfectant liquid outlet.

The gas and the disinfectant liquid may be fed upstream of the rotor, and the gas and the disinfectant liquid may exit downstream of the rotor in one embodiment. Alternatively, the gas is fed downstream of the rotor, the disinfectant liquid is fed upstream of the rotor, and the gas and disinfectant liquid exit downstream of the rotor.

The gas may be transmitted by gas transmitting means, such as a propeller, located opposite to the gas inlet relative to the rotor, and the inlet gas may be pressurized to a pressure of between 1 and 20 bar, more preferably of between 1 and 10 bar.

A preferred embodiment of the invention provides a method wherein a minimum rotation rate $\Omega_{min}$ (rad/s) of the rotor 17 is chosen to satisfy the following relation

$$\Omega_{min} = [\rho_G / (\rho_L d_c R)]^{1/2} u_G \qquad (1)$$

wherein $\Omega_{min}$ is the minimum rotation rate (in radians per sec) of the rotor, $\rho_G$ represents the density of the gas (in kg/m$^3$), $\rho_L$ is the density of the disinfectant liquid (in kg/m$^3$), $d_c$ is channel height (in m), R is the radial position of the channel in the rotor (in m), and $u_G$ is the velocity of the gas (in m/s). In this embodiment, the rotation rate (in radians per sec) $\Omega$ of the rotor is selected such that $\Omega \geq \Omega_{min}$.

The disinfectant liquid is collected before exiting it through the disinfectant liquid outlet to a suitable storage. In this embodiment, the collected disinfected particles are allowed to settle to form a disinfected particle-rich bottom layer and a disinfected particle-poor top layer in the collector that is part of each rotational disinfectant device.

Disinfectant liquid from the disinfected particle-poor top layer is pumped through a return conduit that connects the disinfectant liquid collector outlet with the disinfectant liquid inlet to the disinfectant liquid inlet for re-use. The return conduit, the liquid collector outlet and the disinfectant liquid inlet form a closed fluid circuit. The disinfected particle-rich bottom layer in another embodiment may be drained from the disinfectant liquid collector to an outlet for the disinfected particle-rich bottom layer, which outlet is optionally shut-off after draining.

They may also be transferred to a common storage, if desired. An important advantage of the method is that the infectant has been killed in the short time that it resided in the rotor and came into contact with the disinfectant liquid. Storage of the disinfectant liquid after exiting the device therefore does not pose any problem and the liquid may even be re-used as disinfectant liquid for addition to subsequent rotational disinfectant devices. In this way, a relatively closed assembly of interconnected rotational disinfectant devices may be provided. The disinfectant liquid may be pumped into each rotational disinfectant device separately, but may also be pumped from a common vessel for disinfectant liquid to each rotational disinfectant device through interconnecting piping. Collected disinfected particles may settle in each disinfectant liquid collector of each rotational disinfectant device, or they may settle in storage and/or common vessel such that they remain substantially separated from disinfectant liquid, that is optionally re-used from each collector, storage or common vessel.

Each rotational disinfectant device is preferably positioned in proximity of a source of infectant particles, such as a breathing human. When an assembly of rotational disinfectant devices is used for capturing and disinfecting infectants such as SARS-CoV-2 from air in an indoor space, such as a restaurant or a public transport carriage, wherein the indoor space is provided with a plurality of seats, each seat is preferably associated with a rotational disinfectant device. Alternatively, the air is withdrawn from each source and subsequently joined and filtered by the invented rotational disinfecting device.

BRIEF DESCRIPTION OF THE FIGURES

The above brief description, as well as other objects, features and advantages of the present invention will be more fully appreciated by reference to the following detailed description of presently preferred, but nonetheless illustrative embodiments, when taken in conjunction with the accompanying drawings wherein:

Fig. 1A is a schematic top view of a rotating disinfectant device according to an embodiment of the present invention;
Fig. 1B is a schematic cross-sectional side view of a cross-section along a plane A-A of the embodiment shown in figure 1A;
Fig. 2 is a cross-sectional side view of another embodiment of the rotating disinfectant device according to the invention,
Fig. 3 is a schematic side view of an assembly of rotating disinfectant devices according to an embodiment of the invention,
Fig. 4A is a schematic top view of a rotating disinfectant device according to another embodiment of the present invention;
Fig. 4B is a schematic cross-sectional side view of a cross-section along a plane A-A of the embodiment shown in figure 4A;
Fig. 5A is a schematic top view of a rotating disinfectant device according to another embodiment of the present invention;
Fig. 5B is a schematic cross-sectional side view of a cross-section along a plane A-A of the embodiment shown in figure 5A;
Fig. 6A is a schematic top view of a rotating disinfectant device according to yet another embodiment of the present invention;
Fig. 6B is a schematic cross-sectional side view of a cross-section along a plane A-A of the embodiment shown in figure 6A; and
Fig. 7 schematically shows how an infectant particle is separated and disinfected in a channel of a rotational disinfectant device.

DETAILED DISCLOSURE OF THE INVENTION

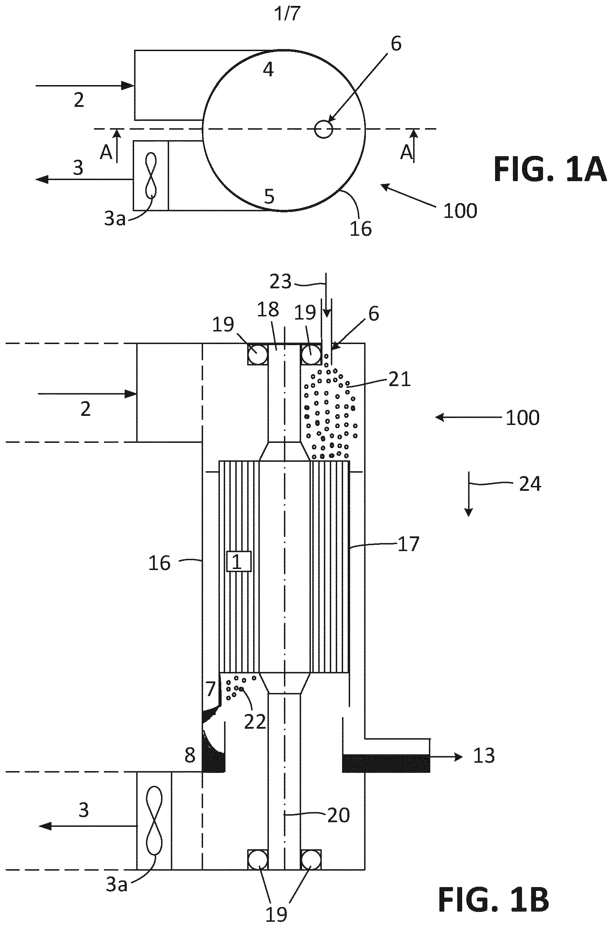

Referring to figures 1A and 1B, a rotating disinfectant device 100 according to an embodiment of the invention is shown. The device 100 comprises a cylindrical housing 16 in which a rotor 17 is mounted on a shaft 18 supported by bearings 19. The rotor 17 consists of a large number of axially extending disinfecting channels 1, arranged parallel to a rotation axis 20.

The rotor 17 is fixed to the shaft 18 which is rotatably mounted in the bearings 19, and which can be externally driven, if desired. Possible leakage between rotor 17 and housing 16 may be prevented by a suitable sealing arrangement. The housing 16 is provided with an air inlet 2 and an air outlet 3. The air inlet 2 consists of a duct that is tangentially positioned at position 4 in the cylindrical housing 16 at an upstream end thereof to induce a swirling rotational motion of the incoming air in the housing 16. The swirling air motion induces a rotation of the rotor 17 within the housing 16 without any external driving means such as a rotational motor. The device further is provided with means for transmitting the virus-containing air from the surroundings to the air inlet 2. A preferred embodiment as shown provides a transmitting means in the form of a propeller 3a or other air suctioning means in the air outlet 3. This transmitting means is essential in being able to suck in virus-containing air into the device 100.

The air outlet configuration 3 is a mirror image of the air inlet configuration 2 and comprises a duct that is tangentially positioned at position 5 in the cylindrical housing 16 at a downstream end thereof to direct the outcoming air from a swirling rotational motion into a translating motion while leaving the housing 16 at position 5.

Apart from the tangential inlets 2 and outlets 3, rotational air motion can also be generated and nullified by stationary curved blades (not shown) provided at an upstream end and a downstream end of the rotor 17. The inner part of the static blade construction may contain the bearings 19 of the rotor 17.

On top of the housing 16 at the upstream end thereof, an inlet 6 for (fresh) disinfectant liquid 21 is provided. In the embodiment shown, the liquid 21 is sprayed on top of the rotor 17 according to arrow 23 by a suitable spraying arrangement. The liquid 21 is sprayed on top of the rotor 16 whereby a rotation of the rotor around the axis 20 provides for an even distribution of liquid 21 over the channels 1. At the downstream side of the rotor 16, the disinfectant liquid 21 that now contains disinfected virus particles (denoted as disinfected virus-containing liquid 22) leaves the facing wall of a cylindrical shell 7 that forms an axial extension of the outer boundary wall of the rotor 16. At an inner side of the cylindrical shell 7 wall, a new film of disinfected virus-containing liquid 22 forms that breaks up at an outer end of the cylindrical shell 7 wall and is propelled as droplets to a liquid collection chamber 8 in the form of a cylindrical ridge provided inside the housing 17. The disinfectant liquid leaves the housing 16 at outlet 13.

As an alternative to the vertical arrangement shown in figures 1A and 1B, the rotational disinfectant device 100 according to an embodiment may be positioned horizontally. The flow of liquid 21 inside the channels 1 is controlled by centrifugal forces and shear forces exerted by the air flowing inside the channels 1 of the rotor 17. When leaving the channels 1 of the rotor 17, the motion of the disinfected virus-containing liquid 22 and liquid droplets is governed by centrifugal forces. Gravitation becomes important only when the liquid flow is brought to rest in collection tanks (not shown) provided outside the rotational disinfectant device.

An external drive or engine may be used when there is a need to rotate the rotor 17 independently of the air flow. Such means for rotating the rotor may in such an embodiment be connected to the rotor 17 through a magnetic coupling for instance. No shaft piercing through the housing 16 is needed in such embodiment, thus keeping the advantage of preventing the use of complicated sealing arrangements to prevent escape of virus-containing air.

Because of the small cross-sectional width of the channels 1, the air will exert a rather strong shear force on the liquid 21 while travelling down the rotor 17 from the upstream end to the downstream end. This may cause the liquid 21 to flow in the same (downstream) direction 24 as the air.

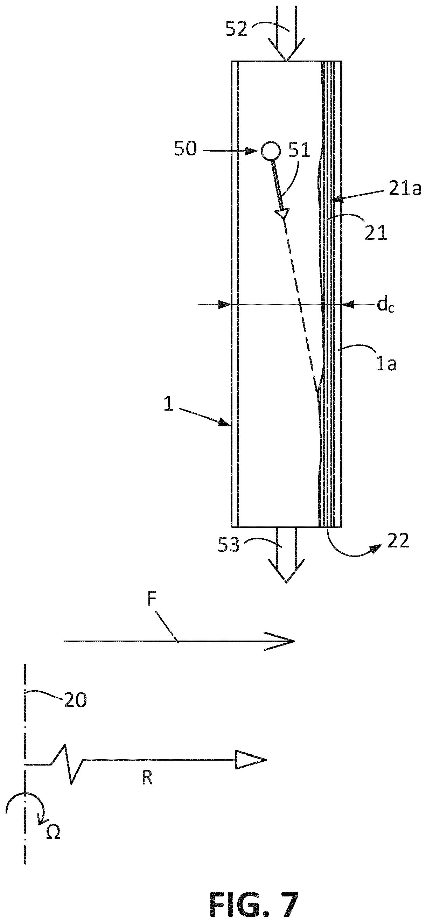

Figure 7 schematically shows the principal working of separation and disinfection inside a channel 1 of the rotational disinfectant device 100. An air flow 52 of average velocity $u_G$ enters a channel 1 of the rotor 17, and exits the channel 1 again at the bottom according to the arrow 53. Rotation of the rotor 17 around the rotation axis 20 of the rotor 17 serves two purposes. Firstly, the centrifugal force F produced by the rotation may force and infectant particle 50 within a channel 1 to move to the radially inward facing wall 1a of the channel 1 according to a trajectory 51, and to arrive inside a film 21a of disinfectant liquid that is formed on the inner facing wall 1a. The disinfectant liquid 21 disinfects the particle 50. Secondly, the centrifugal force F is instrumental in keeping the disinfectant liquid film 21a attached to the inward facing wall 1a. For disinfecting purposes, it is advantageous to keep the liquid film 21a against the wall 1a. To achieve this, a minimum rotational speed $\Omega_{min}$ below which the disinfectant liquid (21, 22) will be blown away from the wall 1a by the moving gas or air may be defined. It is known that this happens for liquid films at the bottom of planar horizontal channels whenever the gas flow becomes too strong in comparison with the gravitational force or gravitational acceleration 'g'. That is, when the gas velocity $u_G$ is larger than $[g.(\rho_L / \rho_G).d_c]^{1/2}$ the liquid film is blown away from the bottom.

In the rotational disinfectant device 100, the centrifugal acceleration $\Omega^2 R$ is preferably designed to be much larger than the gravitational acceleration and sufficiently large to keep the liquid film 21a firmly attached to the wall 1a. To ensure attachment of the liquid film 21a to the inner facing wall 1a and provide an optimum disinfection, the rotation rate $\Omega$ (in rad/s) of the rotor 17 is preferably selected such that $\Omega \geq \Omega_{min}$ wherein:

$$\Omega_{min} = [\rho_G / (\rho_L\, d_c\, R)]^{1/2}\, u_G \qquad (1)$$

wherein $\Omega$ is the rotation rate (radians per sec) of the rotor, $\rho_G$ represents the density of the air (gas) (in kg/m$^3$), $\rho_L$ is the density of the disinfectant liquid (in kg/m$^3$), $d_c$ is channel height (in m), R is the radial position of the channel in the rotor (in m), and $u_G$ is the velocity of the gas (in m/s). The expression tells that a higher gas velocity (a higher capacity or gas intake of the system) also preferably requires a higher rotation rate $\Omega$. Also, the channels 1 positioned at the smallest radial location R are most vulnerable to separation of the liquid film 21a from the wall 1a. The gas velocity is determined by the gas flow $\phi_G$ (in $m^3/s$) through the entire rotor 17, the number 'n' of channels 1 in the rotor 17 and the cross-sectional area $A_c$ (in $m^2$) of the channels, as follows:

$$u_G = \phi_G \, / \, (n \, A_c) \hspace{4cm} (2)$$

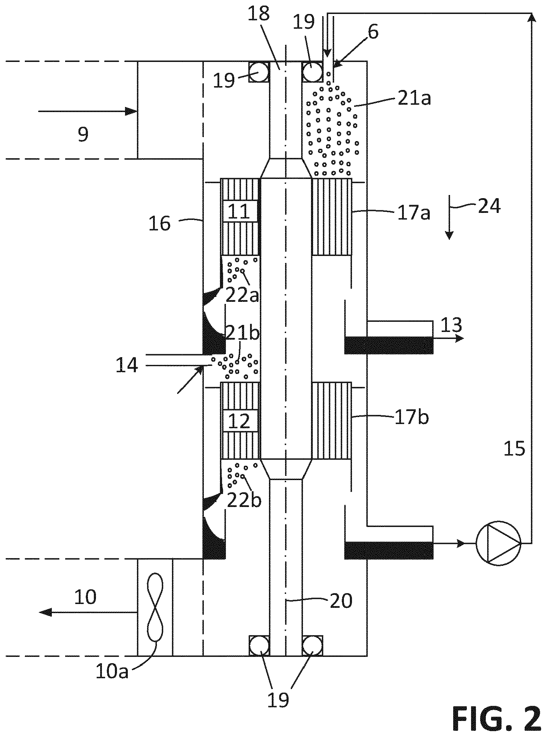

To improve the efficiency of the disinfecting operation, and referring to figure 2, an embodiment of the rotational disinfectant device 100 may comprise a first disinfectant liquid inlet 6 for disinfectant liquid 21a, and a second disinfectant liquid inlet 14 for disinfectant liquid 21b, located upstream of a first rotor 17a and a second rotor 17b respectively, both provided on a common shaft 18. A first disinfectant liquid outlet 13 is provided at a downstream end of the first rotor 17a, whereas a second disinfectant liquid outlet 15 is provided downstream of the second rotor 17b, which outlet 15 reconnects to the first inlet 6. The air enters the device 100 tangentially at the top of the device 100 at inlet 9, and leaves the device 100 at the bottom through outlet 10. The rotating rotors 17a and 17b have been mounted on a common shaft 18 at a top end 11 and a bottom end 12. The rotors 17a and 17b are kept at an axial distance from each other to enable the provision of outlets 13 for removing liquid exiting from the first rotor 17a, and to inject fresh liquid 21b trough inlets 14 to an upstream end of the second rotor 17b. The air exiting the first rotor 17a enters the second rotor 17b, while fresh disinfectant liquid 21b is fed through inlet 14 to the second rotor 17b. When leaving the second rotor 17b, the partly used disinfectant liquid is returned to the first rotor 17a and leaves this rotor 17a as more completely or even fully utilized disinfectant liquid 22b.

The amount of rotors mounted in series on an optionally common shaft may be extended to more than two in order to approximate the configuration of countercurrent absorption in more detail. It is possible therefore to adopt a rotational disinfectant device that comprises at least two rotors in series, more preferably at least three rotors in series, and even more preferably at least five rotors in series.

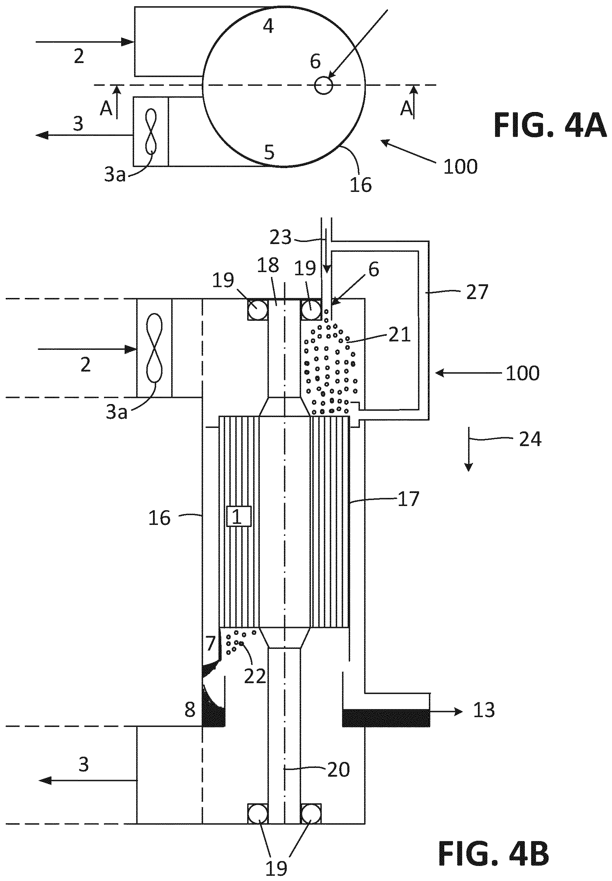

Referring to figures 4A and 4B, a rotating disinfectant device 100 according to yet another embodiment of the invention is shown. The device 100 is similar to the embodiment shown in figures 1A and 1B except for two differences. First, the housing 16 is provided with an air inlet 2 that consists of a duct, tangentially positioned at position 4 in the cylindrical housing 16 at a downstream end thereof to induce a swirling rotational motion of the incoming air in the housing 16. The air outlet configuration 3 is a mirror image of the air inlet configuration 2 and comprises a duct that is tangentially positioned at position 5 in the cylindrical housing 16 at an upstream end thereof to direct the outcoming air from a swirling rotational motion into a translating motion while leaving the housing 16 at position 5. A transmitting means in the form of a propeller 3a or other air suctioning means is provided in the outlet 3.

As with the embodiment of figures 1A and 1B, an inlet 6 for (fresh) disinfectant liquid 21 is provided on top of the housing 16 at the upstream end thereof. At the downstream side of the rotor 16, the disinfectant liquid 21 that now contains virus particles (denoted as virus-containing liquid 22) leaves the facing wall of a cylindrical shell 7 that forms an axial extension of the outer boundary wall of the rotor 16. At an inner side of the cylindrical shell 7 wall, a new film of virus-containing liquid 22 forms that breaks up at an outer end of the cylindrical shell 7 wall and is propelled as droplets to a liquid collection chamber 8 in the form of a cylindrical ridge provided inside the housing 17. The disinfectant liquid leaves the housing 16 at outlet 13.

The embodiment of figures 4A and 4B allows a counter-current operation of the disinfecting device by injecting disinfectant liquid at the top (upstream end) of the device which flows downward while air which is injected at the bottom (downstream end) of the device flows upward. The propeller or fan 3 provides a pressure difference to assist the air flow through the channels. Cleaned air leaves the rotating element at the top and liquid loaded with killed virus particles leaves at the bottom.

A second difference between the embodiment of figures 1A and 1B, and that of figures 4A and 4B is that the latter comprises a return conduit 27 for overflow of disinfectant liquid. The return conduit 27 connects to the housing 16 at an upward end of the rotor 17 and to the inlet 6 provided on top of the housing 16 at the upstream end thereof. Overflowing disinfectant liquid is returned to the inlet 6 through the conduit 27.

Overflow of disinfectant liquid may occur when operating the device in counter-current flow and turbulent conditions, as explained below. Turbulent air flow in the channels will prevail when the Reynolds number is sufficiently large. For practically realistic values of flow velocity and channel diameter, a pressure in the channels of 2, 3, 4 and up to 5 bar may be needed to have a sufficiently large value of the air density to end up with turbulent flow. At increased pressure and density combined with turbulent flow, the shear force acting by the upward streaming air on the liquid film formed in the channels will be large to the extent that air and liquid flow may flow in the same direction, i.e. both upwards. Even when the channels are oriented in a vertical direction, the force of gravity acting on the disinfectant liquid in the channels may not be large enough to result in a downward flow of disinfectant liquid opposite to the upward flow of air. Counter-current flow in the channels may even be impossible in certain cases. On the other hand, at more moderate pressures of below 5 bar for instance, more preferably below 4, bar, even more preferably below 3 bar and most preferably below 2 bar, the upward air flow is prevailingly laminar and the shear force exerted by the upward streaming air on the disinfectant liquid film in the channels may no longer exceed the force of gravity.

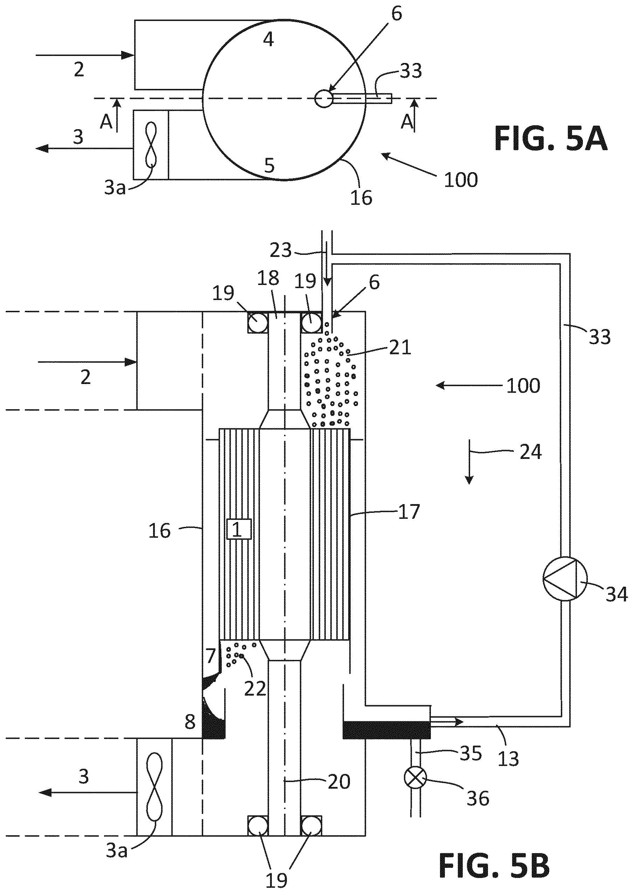

Another embodiment in shown in figures 5A and 5B. This embodiment is similar to the embodiment shown in figures 1A and 1B. However, the embodiment makes use of the fact that in the collector 8, the collected disinfected particles settle to form a disinfected particle-rich bottom layer and a disinfected particle-poor top layer. The embodiment further comprises a return conduit 33 that connects the disinfectant liquid collector outlet 13 with the disinfectant liquid inlet 6, and a pump 34 to transfer disinfectant liquid from the disinfected particle-poor top layer to the disinfectant liquid inlet 6 for re-use.

The rotational disinfectant device according to another embodiment may also comprise an outlet 35 for the disinfected particle-rich bottom layer, optionally provided with a shut-off valve 36. The outlet 35 is provided for draining the disinfected particle-rich bottom layer from the disinfectant liquid collector 8, if needed.

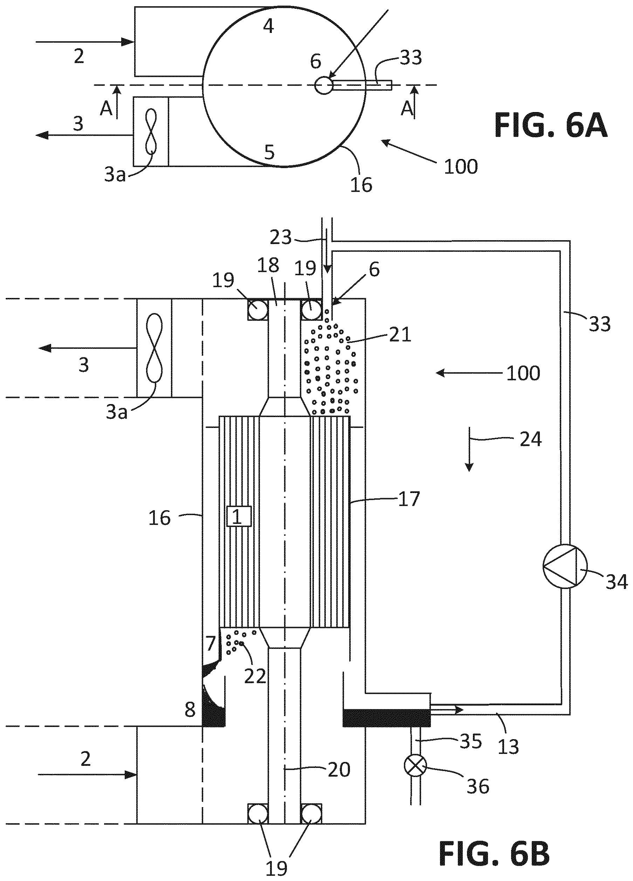

Yet another embodiment in shown in figures 6A and 6B. This embodiment is similar to the embodiment shown in figures 4A and 4B. However, the embodiment makes use of the fact that in the collector 8, the collected disinfected particles settle to form a disinfected particle-rich bottom layer and a disinfected particle-poor top layer. The embodiment further comprises a return conduit 33 that connects the disinfectant liquid collector outlet 13 with the disinfectant liquid inlet 6, and a pump 34 to transfer disinfectant liquid from the disinfected particle-poor top layer to the disinfectant liquid inlet 6 for re-use. This embodiment may also comprise an outlet 35 for the disinfected particle-rich bottom layer, optionally provided with a shut-off valve 36, as disclosed above.

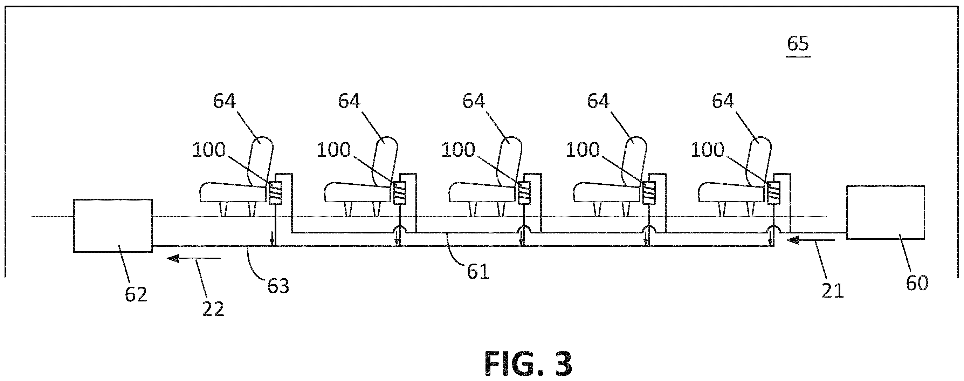

As shown in figure 3, the rotational disinfectant device 100 is particularly useful when used as an assembly of a plurality of such rotational disinfectant devices 100. The assembly comprises a common vessel 60 for distributing disinfectant liquid 21 to each device 100. To this end, the devices 100 each connect to the common vessel 60 for disinfectant liquid 21 via a liquid conduct 61. In the same manner, the assembly comprises a common storage 62 for killed virus-containing disinfectant liquid for collecting virus-containing disinfectant liquid 22 from each device 100. To this end, the devices 100 each connect to the common storage 62 for killed virus-containing disinfectant liquid 22 via a liquid conduct 63. The assembly comprises a pump means (not shown) for pumping the disinfectant liquid (21, 22) to and from each rotational disinfectant device 100.

Each rotational disinfectant device 100 is positioned in proximity of a source of virus particles. In the example of figure 3, the devices 100 are provided in a bus carriage. The indoor space 65 of the bus is provided with a plurality of seats 64, wherein each seat 64 is associated with a rotational disinfectant device 100, as shown in front of each seat 64. The exact position of each device 100 may be different. They may for instance be provided in the front seat back, or under each seat 64. Emitted virus particles are in such an embodiment effectively collected from the air and transmitted and fed to the channels of each rotational disinfectant device, in which channels the virus is killed by contacting it with the disinfectant liquid, in a relatively short time. Alternatively, the virus and bacteria infected air or gas may be withdrawn in close proximity (for instance within a distance of 2 m, preferably 1.5 m, more preferably 1 m) of the source by multiple exhaustion points. The infected air or gas is subsequently transported through tubes, joined and disinfected by a central single rotational disinfecting device, according to an embodiment of the invention.

**Claims**

1. Rotational disinfectant device (100) for capturing and disinfecting infectants, comprising viruses, such as SARS-CoV-2, and bacteria, from a gas, the device (100) comprising means for transmitting the infectant-containing gas to a gas inlet (2) of a housing (16), which housing is further provided with a gas outlet (3), a disinfectant liquid inlet (6) and a disinfectant liquid outlet (13); a rotor (17) mounted for rotation in said housing (16) and connecting to the inlets and outlets, the rotor (17) comprising a plurality of disinfecting channels (1) extending axially and parallel to a common rotation axis (20), wherein the channels are circumferentially enclosed by walls over an, optionally entire, axial length of the rotor (17) between the disinfectant liquid inlet (6) and the disinfectant liquid outlet (13); and wherein the device (100) further comprises means for rotating the rotor (17) and means for providing a sustained flow of disinfectant liquid to the disinfectant liquid inlet (6), wherein the device (100) further comprises

   - a disinfectant liquid collector (8) that connects to the disinfectant liquid outlet (13), in which collector (8) the collected disinfected particles settle to form a disinfected particle-rich bottom layer and a disinfected particle-poor top layer;
   - a return conduit (33) that connects the disinfectant liquid collector outlet (13) with the disinfectant liquid inlet (6), and a pump (34) to transfer disinfectant liquid from the disinfected particle-poor top layer to the disinfectant liquid inlet (6) through the return conduit (33) for re-use;
   wherein the return conduit (33), the liquid collector outlet (13) and the disinfectant liquid inlet (6) form a closed fluid circuit,
wherein the gas transmitting means are preferably located opposite to the gas inlet (2) relative to the rotor (17), and
wherein the gas transmitting means preferably comprise a propeller.

2. Rotational disinfectant device (100) according to claim 1, wherein:

   (a) the gas inlet (2) and the disinfectant inlet (6) are located upstream of the rotor (17), and the gas outlet (3) and the disinfectant liquid outlet (13) are located downstream of the rotor (17); or
   (b) the gas inlet (2) and the disinfectant outlet (13) are located downstream of the rotor (17), while the disinfectant inlet (6) is located upstream of the rotor (17).

3. Rotational disinfectant device (100) according to any one of the preceding claims, wherein the rotor (17) and/or the housing (16) is made from a polymer, such as a polyolefin polymer, preferably a flame retardant polymer.

4. Rotational disinfectant device (100) according to any one of the preceding claims, further comprising a high-efficiency particulate air (HEPA) filter positioned downstream of the rotor (17) of the rotational disinfectant device (100), wherein the high-efficiency particulate air (HEPA) filter is preferably positioned downstream of the disinfectant liquid collector (8) of the rotational disinfectant device (100).

5. Rotational disinfectant device (100) according to any one of the preceding claims, further comprising an outlet (35) for the disinfected particle-rich bottom layer, optionally provided with a shut-off valve (36), which outlet (35) is provided for draining the disinfected particle-rich bottom layer from the disinfectant liquid collector (8).

6. An assembly of a plurality of rotational disinfectant devices (100) according to any one of the preceding claims, wherein the assembly comprises a common vessel (60) for disinfectant liquid, and the devices (100) each connect to the common vessel (60) for disinfectant liquid via a liquid conduct (61), wherein preferably a pump means is provided for pumping the disinfectant liquid to each rotational disinfectant device (100).

7. Indoor space, such as a restaurant or a public transport carriage, provided with an assembly according to claim 6, wherein the indoor space is preferably provided with a plurality of seats, wherein each seat is associated with a rotational disinfectant device, optionally by providing a rotational disinfectant device (100) under each such seat, or in the back of the seat provided in front of a passenger.

8. Method for capturing and disinfecting infectants, comprising viruses, such as SARS-CoV-2, and bacteria, from a gas, the method comprising providing a rotational disinfectant device (100) in accordance with any one of claims 1-5, transmitting and feeding the infectant-containing gas to a gas inlet (2) of the housing (16), feeding a disinfectant liquid to the disinfectant liquid inlet (6), rotating the rotor (17) in said housing (16) which causes the disinfectant liquid to confine to an inward facing wall of the disinfecting channels (1) and form a film thereto, allowing transport of infectant particles from the gas to the disinfectant liquid, and exiting the gas through the gas outlet (3) and the infectant particle-containing disinfectant liquid through the disinfectant liquid outlet (13), wherein the disinfectant liquid is collected before exiting it through the disinfectant liquid outlet (13), and the collected disinfected particles are allowed to settle to form a disinfected particle-rich bottom layer and a disinfected particle-poor top layer, and wherein disinfectant liquid from the disinfected particle-poor top layer is pumped through a return conduit (33) that connects the disinfectant liquid collector outlet (13) with the disinfectant liquid inlet (6) to the disinfectant liquid inlet (6) for re-use, wherein preferably:

   (a) the gas and the disinfectant liquid are fed upstream of the rotor (17), and the gas and the disinfectant liquid exit downstream of the rotor (17); or
   (b) the gas is fed downstream of the rotor (17), the disinfectant liquid is fed upstream of the rotor (17), and the gas and disinfectant liquid exit downstream of the rotor (17).

9. Method according to claim 8, wherein the gas is transmitted by gas transmitting means, such as a propeller, located opposite to the gas inlet (2) relative to the rotor (17).

10. Method according claim 8 or 9, wherein the gas is led through a high-efficiency particulate air (HEPA) filter positioned downstream of the rotor (17) of the rotational disinfectant device (100) before exiting through the gas outlet (3), wherein the gas is preferably led through a high-efficiency particulate air (HEPA) filter positioned downstream of the disinfectant liquid collector (8) of the rotational disinfectant device (100) before exiting through the gas outlet (3).

11. Method according to any one of claims 8-10, wherein the disinfected particle-rich bottom layer is drained from the disinfectant liquid collector (8) to an outlet (35) for the disinfected particle-rich bottom layer, which outlet (35) is optionally shut-off after draining.

12. Method according to any one of claims 8-11, wherein the rotation rate $\Omega$ (in rad/s) of the rotor (17) is chosen such that $\Omega \geq \Omega_{min}$ wherein

$$\Omega_{min} = [\rho_G / (\rho_L \, d_c \, R)]^{1/2} \, u_G \qquad (1)$$

wherein $\Omega$ is the rotation rate (in rad/s) of the rotor (17), $\rho_G$ represents the density of the gas (in kg/m$^3$), $\rho_L$ is the density of the disinfectant liquid (in kg/m$^3$), $d_c$ is channel height (in m), R is the radial position of the channel in the rotor (17) (in m), and $u_G$ is the velocity of the gas (in m/s).

13. Method according to any one of claims 8-12, wherein an assembly in accordance with claim 6 is used, wherein the disinfectant liquid is preferably pumped from the common vessel (60) for disinfectant liquid to each rotational disinfectant device (100).

14. Method according to any one of claims 8-13, provided for capturing and disinfecting infectants, comprising viruses, such as SARS-CoV-2, and bacteria, from a gas in an indoor space, such as a restaurant or a public transport carriage, wherein the indoor space is preferably provided with a plurality of seats, and each seat is associated with a rotational disinfectant device (100).

15. Use of a rotational disinfectant device (100) according to any one of claims 1-5, or an assembly according to claim 6 for capturing and disinfecting infectants, comprising viruses, such as SARS-CoV-2, and bacteria, from a gas.

**Patentansprüche**

1. Rotationsdesinfektionsvorrichtung (100) zum Auffangen und Desinfizieren von Infektiva, umfassend Viren, wie SARS-CoV-2, und Bakterien, aus einem Gas, die Vorrichtung (100) umfassend Mittel zum Übertragen des Infektiva enthaltenden Gases zu einem Gaseinlass (2) eines Gehäuses (16), wobei das Gehäuse ferner mit einem Gasauslass (3), einem Desinfektionsflüssigkeitseinlass (6) und einem Desinfektionsflüssigkeitsauslass (13) versehen ist; einen Rotor (17), der für eine Rotation in dem Gehäuse (16) montiert ist und der mit den Einlässen und Auslässen verbunden ist, der Rotor (17) umfassend eine Vielzahl von Desinfektionskanälen (1), die sich axial und parallel zu einer gemeinsamen Rotationsachse (20) erstreckt, wobei die Kanäle über eine, optional gesamte, axiale Länge des Rotors (17) zwischen dem Desinfektionsflüssigkeitseinlass (6) und dem Desinfektionsflüssigkeitsauslass (13) umfangsmäßig von Wänden umschlossen sind; und wobei die Vorrichtung (100) ferner Mittel zum Rotieren des Rotors (17) und Mittel zum Bereitstellen eines anhaltenden Flusses von Desinfektionsflüssigkeit zu dem Desinfektionsflüssigkeitseinlass (6) umfasst, wobei die Vorrichtung (100) ferner umfasst

   - einen Desinfektionsflüssigkeitssammler (8), der mit dem Desinfektionsflüssigkeitsauslass (13) verbunden ist, wobei sich in dem Sammler (8) die gesammelten desinfizierten Partikel absetzen und eine desinfizierte partikelreiche Bodenschicht und eine desinfizierte partikelarme Oberschicht ausbilden;
   - eine Rücklaufleitung (33), die den Auslass (13) des Desinfektionsflüssigkeitssammlers mit dem Desinfektionsflüssigkeitseinlass (6) verbindet, und eine Pumpe (34), um Desinfektionsflüssigkeit von der desinfizierten partikelarmen Oberschicht durch die Rücklaufleitung (33) hindurch zu dem Desinfektionsflüssigkeitseinlass (6) für eine Wiederverwendung zu übertragen;

     wobei die Rücklaufleitung (33), der Flüssigkeitssammlerauslass (13) und der Desinfektionsflüssigkeitseinlass (6) einen geschlossenen Fluidkreislauf ausbilden,
     wobei sich die Gasübertragungsmittel vorzugsweise gegenüber dem Gaseinlass (2) relativ zu dem Rotor (17) befinden, und
     wobei die Gasübertragungsmittel vorzugsweise einen Propeller umfassen.

2. Rotationsdesinfektionsvorrichtung (100) nach Anspruch 1, wobei:

(a) der Gaseinlass (2) und der Desinfektionsmitteleinlass (6) sich stromaufwärts des Rotors (17) befinden, und der Gasauslass (3) und der Desinfektionsflüssigkeitsauslass (13) sich stromabwärts des Rotors (17) befinden; oder

(b) der Gaseinlass (2) und der Desinfektionsmittelauslass (13) sich stromabwärts des Rotors (17) befinden, während sich der Desinfektionsmitteleinlass (6) stromaufwärts des Rotors (17) befindet.

3. Rotationsdesinfektionsvorrichtung (100) nach einem der vorstehenden Ansprüche, wobei der Rotor (17) und/oder das Gehäuse (16) aus einem Polymer, wie einem Polyolefinpolymer, vorzugsweise einem flammhemmenden Polymer, hergestellt ist.

4. Rotationsdesinfektionsvorrichtung (100) nach einem der vorstehenden Ansprüche, ferner umfassend einen hocheffizienten Partikelluftfilter (HEPA-Filter), der stromabwärts des Rotors (17) der Rotationsdesinfektionsvorrichtung (100) positioniert ist, wobei der hocheffiziente Partikelluftfilter (HEPA-Filter) vorzugsweise stromabwärts des Desinfektionsflüssigkeitssammlers (8) der Rotationsdesinfektionsvorrichtung (100) positioniert ist.

5. Rotationsdesinfektionsvorrichtung (100) nach einem der vorstehenden Ansprüche, ferner umfassend einen Auslass (35) für die desinfizierte partikelreiche Bodenschicht, der optional mit einem Absperrventil (36) versehen ist, wobei der Auslass (35) zum Ablassen der desinfizierten partikelreichen Bodenschicht aus dem Desinfektionsflüssigkeitssammler (8) bereitgestellt ist.

6. Anordnung aus einer Vielzahl von Rotationsdesinfektionsvorrichtungen (100) nach einem der vorstehenden Ansprüche, wobei die Anordnung ein gemeinsamer Behälter (60) für die Desinfektionsflüssigkeit umfasst und die Vorrichtungen (100) jeweils über eine Flüssigkeitsleitung (61) mit dem gemeinsamen Behälter (60) für die Desinfektionsflüssigkeit verbunden sind, wobei vorzugsweise ein Pumpmittel zum Pumpen der Desinfektionsflüssigkeit zu jeder Rotationsdesinfektionsvorrichtung (100) bereitgestellt ist.

7. Innenraum, wie ein Restaurant oder ein Wagen eines öffentlichen Verkehrsmittels, der mit einer Anordnung nach Anspruch 6 versehen ist, wobei der Innenraum vorzugsweise mit einer Vielzahl von Sitzen versehen ist, wobei jedem Sitz eine Rotationsdesinfektionsvorrichtung zugeordnet ist, optional durch Bereitstellen einer Rotationsdesinfektionsvorrichtung (100) unter jedem dieser Sitze oder an der Rückseite des Sitzes, der vor einem Passagier bereitgestellt ist.

8. Verfahren zum Auffangen und Desinfizieren von Infektiva, umfassend Viren wie SARS-CoV-2 und Bakterien, aus einem Gas, das Verfahren umfassend das Bereitstellen einer Rotationsdesinfektionsvorrichtung (100) nach einem der Ansprüche 1 bis 5, das Übertragen und das Zuführen des Infektiva enthaltenden Gases zu einem Gaseinlass (2) des Gehäuses (16), das Zuführen einer Desinfektionsflüssigkeit zu dem Desinfektionsflüssigkeitseinlass (6), das Rotieren des Rotors (17) in dem Gehäuse (16), das die Desinfektionsflüssigkeit veranlasst, an einer nach innen gerichteten Wand der Desinfektionskanäle (1) eingeschlossen zu werden, und einen Film darauf ausbildet, was den Transport von Infektivapartikeln aus dem Gas zu der Desinfektionsflüssigkeit ermöglicht, und das Austreten des Gases durch den Gasauslass (3) hindurch und der Infektivapartikel enthaltenden Desinfektionsflüssigkeit durch den Desinfektionsflüssigkeitsauslass (13) hindurch, wobei die Desinfektionsflüssigkeit gesammelt wird, bevor sie durch den Desinfektionsflüssigkeitsauslass (13) hindurch austritt, und den gesammelten desinfizierten Partikeln ermöglicht wird, sich abzusetzen, um eine desinfizierte partikelreiche Bodenschicht und eine desinfizierte partikelarme Oberschicht auszubilden, und wobei Desinfektionsflüssigkeit aus der desinfizierten partikelarmen Oberschicht durch eine Rücklaufleitung (33) hindurch, die den Auslass (13) des Desinfektionsflüssigkeitssammlers mit dem Desinfektionsflüssigkeitseinlass (6) verbindet, zu dem Desinfektionsflüssigkeitseinlass (6) für die Wiederverwendung gepumpt wird, wobei vorzugsweise:

(a) das Gas und die Desinfektionsflüssigkeit stromaufwärts des Rotors (17) zugeführt werden, und das Gas und die Desinfektionsflüssigkeit stromabwärts des Rotors (17) austreten; oder

(b) das Gas stromabwärts des Rotors (17) zugeführt wird, die Desinfektionsflüssigkeit stromaufwärts des Rotors (17) zugeführt wird, und das Gas und die Desinfektionsflüssigkeit stromabwärts des Rotors (17) austreten.

9. Verfahren nach Anspruch 8, wobei das Gas durch ein Gasübertragungsmittel, wie einen Propeller, übertragen wird, die sich relativ zu dem Rotor (17) gegenüber dem Gaseinlass (2) befinden.

10. Verfahren nach Anspruch 8 oder 9, wobei das Gas durch einen hocheffizienten Partikelluftfilter (HEPA) hindurch geleitet wird, der stromabwärts des Rotors (17) der Rotationsdesinfektionsvorrichtung (100) positioniert ist, bevor es durch den Gasauslass (3) hindurch austritt, wobei das Gas vorzugsweise durch einen hocheffizienten Partikelluftfilter (HEPA) hindurch geleitet wird, der stromabwärts des Desinfektionsflüssigkeitssammlers (8) der Rotationsdesinfektionsvorrichtung (100) positioniert ist, bevor es durch den Gasauslass (3) hindurch austritt.

11. Verfahren nach einem der Ansprüche 8 bis 10, wobei die desinfizierte partikelreiche Bodenschicht aus dem Desinfektionsflüssigkeitssammler (8) zu einem Auslass (35) für die desinfizierte, partikelreiche Bodenschicht abgelassen wird, wobei der Auslass (35) nach dem Ablassen optional abgesperrt wird.

12. Verfahren nach einem der Ansprüche 8 bis 11, wobei die Rotationsgeschwindigkeit $\Omega$ (in rad/s) des Rotors (17) derart gewählt wird, dass $\Omega \geq \Omega_{min}$, wobei

$$\Omega_{min} = [\rho_G/(\rho_L\, d_c\, R)]^{1/2} u_G \quad (1)$$

wobei $\Omega$ die Rotationsgeschwindigkeit (in rad/s) des Rotors (17) ist, $\rho_G$ die Dichte des Gases (in kg/m$^3$) darstellt, $\rho_L$ die Dichte der Desinfektionsflüssigkeit (in kg/m$^3$) ist, $d_c$ die Kanalhöhe (in m) ist, R die radiale Position des Kanals in dem Rotor (17) (in m) ist und $u_G$ die Geschwindigkeit des Gases (in m/s) ist/

13. Verfahren nach einem der Ansprüche 8 bis 12, wobei eine Anordnung nach Anspruch 6 verwendet wird, wobei die Desinfektionsflüssigkeit vorzugsweise aus dem gemeinsamen Behälter (60) für Desinfektionsflüssigkeit zu jeder Rotationsdesinfektionsvorrichtung (100) gepumpt wird.

14. Verfahren nach einem der Ansprüche 8 bis 13, zum Auffangen und Desinfizieren von Infektiva, umfassend Viren, wie SARS-CoV-2, und Bakterien, aus einem Gas in einem Innenraum, wie einem Restaurant oder einem Wagen eines öffentlichen Verkehrsmittels, wobei der Innenraum vorzugsweise mit einer Vielzahl von Sitzen versehen ist und jeder Sitz einer Rotationsdesinfektionsvorrichtung (100) zugeordnet ist.

15. Verwendung einer Rotationsdesinfektionsvorrichtung (100) nach einem der Ansprüche 1 bis 5 oder einer Anordnung nach Anspruch 6 zum Auffangen und Desinfizieren von Infektiva, umfassend Viren, wie SARS-CoV-2, und Bakterien, aus einem Gas.


**Revendications**

1. Dispositif (100) de désinfection rotatif permettant de capturer et de désinfecter des agents infectieux, comprenant des virus, tels que le SARS-CoV-2, et des bactéries, à partir d'un gaz, le dispositif (100) comprenant des moyens pour transmettre le gaz contenant des agents infectieux à une entrée de gaz (2) d'un boîtier (16), lequel boîtier est en outre pourvu d'une sortie de gaz (3), d'une entrée de liquide désinfectant (6) et d'une sortie de liquide désinfectant (13) ; un rotor (17) monté pour tourner dans ledit boîtier (16) et relié aux entrées et sorties, le rotor (17) comprenant une pluralité de canaux de désinfection (1) s'étendant axialement et parallèlement à un axe de rotation commun (20), les canaux étant enfermés circonférentiellement par des parois sur une longueur axiale, éventuellement totale, du rotor (17) entre l'entrée de liquide désinfectant (6) et la sortie de liquide désinfectant (13) ; et dans lequel le dispositif (100) comprend en outre des moyens pour faire tourner le rotor (17) et des moyens pour fournir un écoulement soutenu de liquide désinfectant à l'entrée de liquide désinfectant (6), le dispositif (100) comprenant en outre

   - un collecteur (8) de liquide désinfectant relié à la sortie de liquide désinfectant (13), collecteur (8) dans lequel les particules désinfectées collectées se déposent pour former une couche inférieure riche en particules désinfectées et une couche supérieure pauvre en particules désinfectées ;
   - un conduit de retour (33) qui relie la sortie de collecteur de liquide désinfectant (13) à l'entrée de liquide désinfectant (6), et une pompe (34) pour transférer du liquide désinfectant de la couche supérieure pauvre en particules désinfectées à l'entrée de liquide désinfectant (6) à travers le conduit de retour (33) en vue d'une réutilisation ;

      dans lequel le conduit de retour (33), la sortie de collecteur de liquide (13) et l'entrée de liquide désinfectant (6) forment un circuit de fluide fermé,
      dans lequel les moyens de transmission de gaz sont de préférence situés à l'opposé de l'entrée de gaz (2) par rapport au rotor (17), et

dans lequel les moyens de transmission de gaz comprennent de préférence une hélice.

2. Dispositif (100) de désinfection rotatif selon la revendication 1, dans lequel :

(a) l'entrée de gaz (2) et l'entrée de désinfectant (6) sont situées en amont du rotor (17), et la sortie de gaz (3) et la sortie de liquide désinfectant (13) sont situées en aval du rotor (17) ; ou
(b) l'entrée de gaz (2) et la sortie de désinfectant (13) sont situées en aval du rotor (17), tandis que l'entrée de désinfectant (6) est située en amont du rotor (17).

3. Dispositif (100) de désinfection rotatif selon l'une quelconque des revendications précédentes, dans lequel le rotor (17) et/ou le boîtier (16) est constitué d'un polymère, tel qu'un polymère de polyoléfine, de préférence un polymère ignifuge.

4. Dispositif (100) de désinfection rotatif selon l'une quelconque des revendications précédentes, comprenant en outre un filtre à particules à haute efficacité (HEPA) placé en aval du rotor (17) du dispositif (100) de désinfection rotatif, le filtre à particules à haute efficacité (HEPA) étant de préférence placé en aval du collecteur (8) de liquide désinfectant du dispositif (100) de désinfection rotatif

5. Dispositif (100) de désinfection rotatif selon l'une quelconque des revendications précédentes, comprenant en outre une sortie (35) pour la couche inférieure riche en particules désinfectées, éventuellement munie d'une vanne d'arrêt (36), laquelle sortie (35) étant prévue pour évacuer la couche inférieure riche en particules désinfectées du collecteur (8) de liquide désinfectant.

6. Ensemble d'une pluralité de dispositifs (100) de désinfection rotatifs selon l'une quelconque des revendications précédentes, dans lequel l'ensemble comprend un récipient commun (60) pour liquide désinfectant, et les dispositifs (100) sont reliés chacun au récipient commun (60) pour liquide désinfectant par l'intermédiaire d'un conduit de liquide (61), dans lequel de préférence un moyen de pompage est prévu pour pomper le liquide désinfectant vers chaque dispositif (100) de désinfection rotatif.

7. Espace intérieur, tel qu'un restaurant ou une voiture de transport public, pourvu d'un ensemble selon la revendication 6, dans lequel l'espace intérieur est de préférence pourvu d'une pluralité de sièges, chaque siège étant associé à un dispositif de désinfection rotatif, éventuellement en prévoyant un dispositif (100) de désinfection rotatif sous chacun des tels sièges, ou à l'arrière du siège situé devant un passager.

8. Procédé de capture et de désinfection d'agents infectieux, comprenant des virus, tels que le SARS-CoV-2, et des bactéries, à partir d'un gaz, le procédé comprenant la fourniture d'un dispositif (100) de désinfection rotatif selon l'une quelconque des revendications 1 à 5, la transmission et l'acheminement du gaz contenant des agents infectieux à une entrée de gaz (2) du boîtier (16), l'acheminement d'un liquide désinfectant vers l'entrée de liquide désinfectant (6), la rotation du rotor (17) dans ledit boîtier (16), ce qui amène le liquide désinfectant à se confiner sur une paroi orientée vers l'intérieur des canaux de désinfection (1) et à former un film sur celle-ci, le fait de permettre le transport des particules d'agents infectieux à partir du gaz vers le liquide désinfectant, et l'évacuation du gaz à travers la sortie de gaz (3) et du liquide désinfectant contenant des particules d'agents infectieux à travers la sortie de liquide désinfectant (13), le liquide désinfectant étant collecté avant d'être évacué à travers la sortie de liquide désinfectant (13), et les particules désinfectées collectées pouvant se déposer pour former une couche inférieure riche en particules désinfectées et une couche supérieure pauvre en particules désinfectées, et dans lequel le liquide désinfectant de la couche supérieure pauvre en particules désinfectées est pompé à travers un conduit de retour (33) qui relie la sortie de collecteur de liquide désinfectant (13) à l'entrée de liquide désinfectant (6) en vue d'une réutilisation, dans lequel de préférence :

(a) le gaz et le liquide désinfectant sont acheminés en amont du rotor (17), et le gaz et le liquide désinfectant sont évacués en aval du rotor (17) ; ou
(b) le gaz est acheminé en aval du rotor (17), le liquide désinfectant est acheminé en amont du rotor (17), et le gaz et le liquide désinfectant sont évacués en aval du rotor (17).

9. Procédé selon la revendication 8, dans lequel le gaz est transmis par des moyens de transmission de gaz, tels qu'une hélice, situés à l'opposé de l'entrée de gaz (2) par rapport au rotor (17).

10. Procédé selon la revendication 8 ou 9, dans lequel le gaz est conduit à travers un filtre à particules à haute efficacité (HEPA) placé en aval du rotor (17) du dispositif (100) de désinfection rotatif avant de s'évacuer à travers la sortie de gaz (3), le gaz étant de préférence conduit à travers un filtre à particules à haute efficacité (HEPA) placé en aval du collecteur (8) de liquide désinfectant du dispositif (100) de désinfection rotatif avant de s'évacuer à travers la sortie de gaz (3).

11. Procédé selon l'une quelconque des revendications 8 à 10, dans lequel la couche inférieure riche en particules désinfectées est évacuée du collecteur (8) de liquide désinfectant vers une sortie (35) pour la couche inférieure riche en particules désinfectées, laquelle sortie (35) étant éventuellement fermée après l'évacuation.

12. Procédé selon l'une quelconque des revendications 8 à 11, dans lequel la vitesse de rotation $\Omega$ (en rad/s) du rotor (17) est choisie de telle sorte que $\Omega \geq \Omega_{\min}$ où

$$\Omega_{\min} = [\rho_G/(\rho_L\, d_c\, R)]^{1/2} u_G \ (1)$$

où $\Omega$ représente la vitesse de rotation (en rad/s) du rotor (17), $\rho_G$ représente la densité du gaz (en kg/m$^3$), $\rho_L$ représente la densité du liquide désinfectant (en kg/m$^3$), $d_c$ représente la hauteur de canal (en m), R représente la position radiale du canal dans le rotor (17) (en m), et $u_G$ représente la vitesse du gaz (en m/s).

13. Procédé selon l'une quelconque des revendications 8 à 12, dans lequel un ensemble selon la revendication 6 est utilisé, le liquide désinfectant étant de préférence pompé du récipient commun (60) pour liquide désinfectant vers chaque dispositif (100) de désinfection rotatif.

14. Procédé selon l'une quelconque des revendications 8 à 13, permettant de capturer et de désinfecter des agents infectieux, comprenant des virus, tels que le SARS-CoV-2, et des bactéries, à partir d'un gaz dans un espace intérieur, tel qu'un restaurant ou une voiture de transport public, dans lequel l'espace intérieur est de préférence pourvu d'une pluralité de sièges, et chaque siège est associé à un dispositif (100) de désinfection rotatif.

15. Utilisation d'un dispositif (100) de désinfection rotatif selon l'une quelconque des revendications 1 à 5, ou d'un ensemble selon la revendication 6, permettant de capturer et de désinfecter des agents infectieux, comprenant des virus, tels que le SARS-CoV-2, et des bactéries, à partir d'un gaz.

**FIG. 1A**

**FIG. 1B**

**FIG. 2**

FIG. 3

**FIG. 4A**

**FIG. 4B**

**FIG. 5A**

**FIG. 5B**

FIG. 6A

FIG. 6B

**FIG. 7**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3624923 A1